# EUROPÄISCHE PATENTSCHRIFT

(11) **EP 0 359 079 B1**
(45) Veröffentlichungstag und Bekanntmachung des Hinweises auf die Patenterteilung: **03.02.1993**
(21) Anmeldenummer: 89116262.0
(22) Anmeldetag: 02.09.1989
(51) Int. Cl.: A61F 9/00, G02C 13/00, A45C 11/04

(54) **Vorrichtung zum Untersuchen von Kontaktlinsen**
Contact lens inpecting device
Dispositif pour examiner des lentilles de contact

(30) Priorität: 07.09.1988 CH 3350/88
(43) Veröffentlichungstag der Anmeldung: 21.03.1990
(73) Patentinhaber: Bieri, Fritz, Dr. med., CH-8645 Jona (CH)
(72) Erfinder: Bieri, Fritz, Dr. med., CH-8645 Jona (CH)

(56) Entgegenhaltungen:
- GB-A- 2 093 605
- US-A- 4 002 234
- US-A- 4 392 569
- US-A- 4 520 923
- US-A- 4 545 479

## Beschreibung

Die Erfindung betrifft eine Untersuchungsvorrichtung für Kontaktlinsen, mit welcher Ablagerungen auf den Oberflächen und Materialveränderungen der Kontaktlinsen festgestellt werden.

Auf den Oberflächen der Kontaktlinsen bilden sich bekanntlich im Laufe ihrer Benutzung organische und anorganische Ablagerungen und setzen sich Mikroorganismen sowie Pilze fest. Bei den organischen Ablagerungen handelt es sich um Bestandteile (Proteine, Mucine, Lipide) der Tränenflüssigkeit. Die anorganischen Ablagerungen (Metall, Eisen, Rost) rühren von arbeitsbedingten Umständen (Luftverschmutzung, Strassenstaub, Metallabrieb u.s.w.) her. Zu den anorganischen Ablagerungen zählen auch diejenigen von Calciumsalzen, welche auch als Hartwasserablagerungen bezeichnet werden. Sie entstehen infolge Abspülens der Kontaktlinsen mit Leitungswasser oder durch Veränderung des pH-Wertes der Tränenflüssigkeit (Medikamente, physiologische Einflüsse). Die Mikroorganismen (Bakterien, Viren) und Pilze gehören zur normalen Mikroflora der Bindehaut, des Bindehautsackes und der tränenabführenden Wege. Dies ändert sich, sobald das Gleichgewicht der Mikroflora gestört wird, was durch Keimübertragungen von Händen, unsachgemässe Reinigung und Desinfektion, Erkrankung oder Stoffwechselstörungen (Diabetiker) des Linsenträgers oder Berührung der Kontaktlinsen mit der Umwelt geschieht. Ferner ist bekannt, dass die Kontaktlinsen im Laufe ihrer Benutzung Veränderungen und Beschädigungen ihres Materials unterworfen sind. Die Materialveränderungen (z.B. Verfärbungen) beruhen auf Umwelteinflüsse (Zigarettenrauch, Farbdämpfe, Kosmetika, Abfärben von Aufbewahrungsbehältern). Die Beschädigungen (Haarrisse, Abschürfungen, Schrammen, Randausbrüche) beruhen auf unsachgemässer Behandlung der Kontaktlinsen.

Die aufgezählten Veränderungen der Kontaktlinsen bewirken eine Reihe verschiedener Komplikationen für den Benutzer bzw. Träger, wie z.B. eine Unverträglichkeit gegenüber den Kontaktlinsen, die nicht mehr getragen werden können. Auch akute Zustände, wie die Begünstigung von Infekten (besonders oft zu beobachten bei Linsen mit verlängerter Tragedauer) oder chronischer Reizzustände, wie eine gigantopapilläre Conjunctivitis, werden weniger häufig gesehen, stehen dann aber im Zentrum von ärztlichen Bemühungen.

Da die Kontaktlinsen vielen Einwirkungen unterworfen sind, die ihre Tragequalität und ihre optischen Eigenschaften ungünstig beeinflussen, sind regelmässige Kontrollen durch den Anpasser (Augenarzt, Optiker) und Träger vorzusehen. Jeder Träger von Kontaktlinsen wird instruiert, diese möglichst täglich zu reinigen. Je früher die Reinigung erfolgt, desto grösser ist der Erfolg. Verschiedene Reinigungsmöglichkeiten kommen zur Anwendung, wie z.B. chemische, thermische, encymatische, mechanische oder Ultraschall-Reinigungen. Zur Durchführung der Kontrollen bedient man sich folgender Handhabungen:
- Kontaktlinsenbetrachtung auf dem Auge mit einer Spaltlampe (binoculares Mikroskop);
- Kontaktlinsenbetrachtung mit auf dem Finger oder in einem transparenten mit Flüssigkeit gefüllten Behälter mittels blossem Auge und der Spaltlampe;
- Kontaktlinsenbetrachtung in Dunkelfeldbeleuchtung mit einem binocularen Mikroskop.

Solche Betrachtungsweisen sind kompliziert, nur beim Augenarzt oder Optiker möglich oder haben eine ungenügende Aussagekraft.

Ein Aufbewahrungsgefäss für Kontaktlinsen wird im US-A-4 392 569 vorgeschlagen, in welchem zwei Kontaktlinsen so gelagert sind, dass sie mit Flüssigkeit benetzt durch ungewollte Bewegungen des Aufbewahrungsgefässes nicht beschädigt werden. Eine Untersuchung der gelagerten Kontaktlinsen ist nicht möglich.

In US-A-4 545 479 wird ein Aufbewahrungsgefäss für Kontaktlinsen vorgeschlagen, das auch eine Untersuchung derselben erlaubt. Jede der beiden Kontaktlinsen befindet sich zwischen zwei im Aufbewahrungsgefäss angebrachten Sammellinsen. Diese Linsen haben einen bestimmten optischen Mindestabstand zur Kontaktlinse damit der Beobachter ein vergrössertes Bild der im Aufbewahrungsgefäss gelagerten Kontaktlinse sieht. Die Mindestabstände bedingen ein für ein Kontaktlinsen-Aufbewahrungsgefäss unhandliches Volumen. Da die Kontaktlinsen in dem Aufbewahrungsgefäss in unerwünschter Weise frei beweglich sind, kann deren Untersuchung nur in einer bestimmten Stellung des Aufbewahrungsgefässes und bei künstlicher Beleuchtung durchgeführt werden. Die künstliche Beleuchtung ist im Aufbewahrungsgefäss eingebaut und trägt auch zur nachteiligen Volumenvergrösserung desselben bei. Ein weiterer Nachteil ist in den verschiedenen Konstruktionsteilen des Aufbewahrungsgefässes zu sehen, die im optischen Untersuchungsweg liegen und ihre Strukturen in das vergrösserte Bild der Kontaktlinsen projizieren. Der Beobachter kann diese Strukturen von den Ablagerungen und Beschädigungen der Kontaktlinsen nicht unterscheiden.

Die Erfindung hat die Aufgabe, diese Nachteile zu beseitigen und ein einfaches, handliches Betrachtungsgerät zu konstruieren, das sowohl vom Augenarzt und Optiker als auch vom Benutzer mühelos und leicht verwendet werden kann, wobei die Veränderungen der Kontaktlinse und die Ablagerungen auf deren Oberflächen durch verschiedenartige Untersuchungsmethoden schon in ihren Anfängen erkannt werden. Hierdurch werden die oben beschriebenen unerwünschten Einflüsse auf die Kontaktlinsen früher erfasst, so dass ihre Minderungen an Tragekomfort und optischen Eigenschaften beseitigt werden. Problemlinsen werden rechtzeitig erkannt und entweder behandelt oder ersetzt.

Die Aufgabe der Erfindung wird durch die Merkmale des kennzeichnenden Teils des Patentanspruchs 1 gelöst.

Ausführungsbeispiele der Erfindung werden anhand der Zeichnungen näher erläutert. Es zeigen:
Figur 1 einen stabförmigen Körper aus durchsichtigem Material in perspektivischer Darstellung;
Figuren 2,3,4 verschiedene Krümmungen der einen Endfläche des stabförmigen Körpers, welche die Kontaktlinsen aufnimmt;
Figur 5 die Ausgestaltung der anderen Endfläche des stabförmigen Körpers, der aus Elementen zusammensetzbar ist;
Figur 6 eine weitere Ausführung des stabförmigen Körpers mit seinen beiden Endflächen;
Figur 7 einen stabförmigen Körper mit einem Hohlraum.

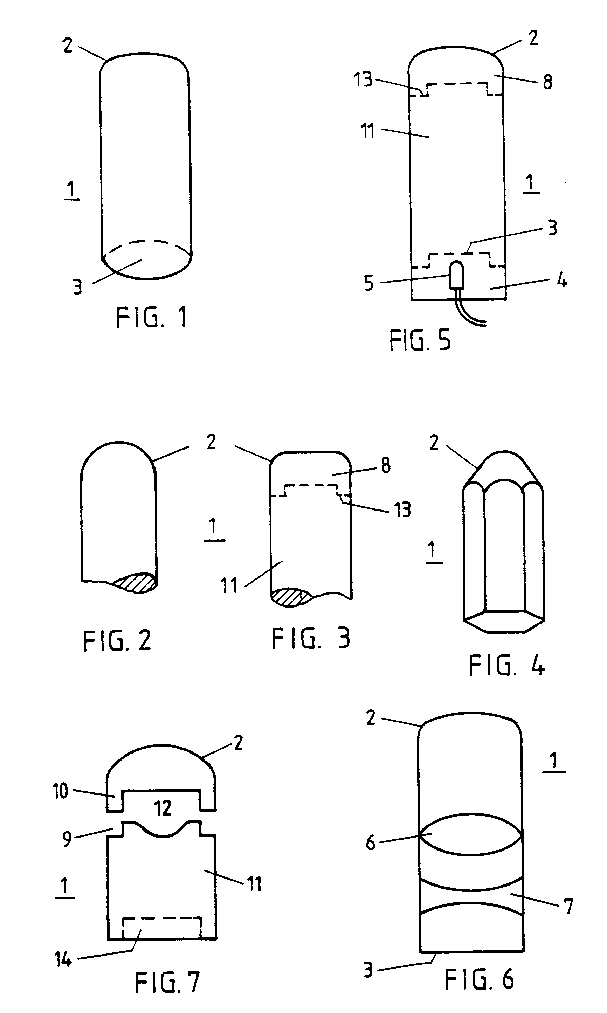

Die Figur 1 zeigt einen transparenten Körper 1 mit glatter Oberfläche aus z.B. Acrylglas, dessen eine Endfläche 2 sphärisch, asphärisch bzw. toroidförmig und dessen andere Endfläche 3 plan ausgebildet sind. Die zu untersuchende Kontaktlinse wird auf die eine Endfläche 2 gelegt. Die Haftung der Kontaktlinse auf der Endfläche 2 erfolgt infolge der Flächenkrümmung, die vorzugsweise aus mehreren von verschiedenen Krümmungsradien erzeugten Krümmungsbereiche besteht. Die Krümmungsradien liegen in der Grössenordnung von 7 mm bis 10,5 mm und umfassen somit die Krümmungen der handelsüblichen Kontaktlinsen. Die Kontaktlinsen aus hydrophilem oder hochhydrophilem Material werden vorzugsweise auf die in die Nähe der Längsachse des Körpers 1 liegende Mittelzone der Endfläche 2 gelegt. Die Kontaktlinsen aus gaspermeablem Material werden vorzugsweise in die Randzone der Endfläche 2 gelegt. In besonderen Fällen kann die Haftung der Kontaktlinsen durch eine auf die Endfläche 2 applizierte Flüssigkeit erhöht werden. Die andere Endfläche 3 ist eben und matt geschliffen. Die auf der sphärischen Endfläche angeordnete Kontaktlinse kann auf zweierlei Arten betrachtet werden. In der normal hellen Umgebung wird die Kontaktlinse gegen einen dunklen Hintergrund betrachtet. Somit wird nur die Kontaktlinse beleuchtet. Oder man richtet die mattierte Endfläche 3 gegen eine Leuchtquelle, so dass die Kontaktlinse in Durchlicht bestrahlt werden kann. Die Leuchtquelle, die das Tageslicht, eine Spaltlampe oder eine Leuchtdiode 5, (Figur 5) sein kann, wird nicht als solche gesehen. Man erblickt nur eine gleichmässig helle Fläche. Der Betrachter kann eine Lupe zur Vergrösserung des Bildes der Kontaktlinse nehmen.

In der Figur 1 ist der transparente Körper 1 zylinderförmig (kreisförmiger Querschnitt) dargestellt. Selbstverständlich kann er stabförmig mit einem elliptischen oder polygonen Querschnitt (z.B. sechseckig) ausgebildet sein. Der elliptische oder polygon gebildete stabförmige Körper ist durch den Benutzer besser zu handhaben. Der Körper muss nicht aus einem Stück bestehen. Er kann auch aus Elementen zusammengesteckt sein, wie das z.B. in Figuren 3,5,7 gezeigt ist. Eine Steckverbindung hat sich in der Herstellung und im Gebrauch als einfach und bequem erwiesen. Grundsätzlich sind auch andere Verbindungsarten möglich.

In den Figuren 2,3,4 sind sphärische Endflächen 2 mit unterschiedlichen Krümmungen dargestellt, die sich vorzüglich zur einwandfreien Auflage der hydrophilen und gaspermeablen Kontaktlinsen eignen. Besonders bei weichen Kontaktlinsen ist zu beachten, dass sie ohne Faltenbildung an der Endfläche 2 anliegen. Die Formen der verschiedenen Endflächen sind annäherungsweise mit der Oberfläche des Augapfels vergleichbar. Sie können sphärisch, torisch oder asphärisch geschliffen sein. Die Figur 2 zeigt eine Krümmung der Endfläche 2, die einen einzigen Krümmungsradius erhält. Die Figur 3 zeigt eine Endfläche 2 mit einer aus zwei unterschiedlichen Krümmungsradien hergestellten Krümmung. Ferner zeigt die Figur 3 die Möglichkeit, den Körper 1 aus Elementen aufzubauen; z.B. kann ein Element 8, das die Endfläche mit der gewünschten Krümmung enthält, mit einem Körperelement 11 zusammengesetzt werden. Dies ist als Steckverbindung 13 gestrichelt gezeichnet. Wenn Figuren 2 + 3 einen Körper bzw. Elemente mit kreisförmigem Querschnitt zeigen, so sei darauf hingewiesen, dass auch elliptische oder polygone Querschnitte vorgesehen sind. Die Figur 4 zeigt eine Endfläche 2, deren Krümmung aus mehreren Krümmungsradien gebildet ist. Ferner ist in der Figur 4 ein Körper 1 mit sechseckigem Querschnitt dargestellt, auch hier besteht die Möglichkeit, den Körper aus mehreren Elementen zusammenzusetzen, was nicht gesondert gezeichnet ist.

Die Figur 5 zeigt den transparenten Körper 1 mit glatter Oberfläche, dessen Endfläche 3 mattiert ist. An der Endfläche ist eine Halterung 4 angebracht, in welcher eine Glühbirne oder eine Leuchtdiode 5 vorgesehen ist, die von einer mitgeführten Batterie oder von der normalen Stromversorgung gespiesen werden. Mit dieser Ausführung ist der Benutzer (Augenarzt, Optiker, Träger) unabhängig von anderen Lichtquellen. Die Halterung 4 kann als Element an den Körper 1 oder an das Körperelement 11 angebracht sein. Im ersten Fall sind die mattierte Endfläche 3 und die Oberfläche der Halterung eben und werden miteinander verbunden, was z.B. durch Kleben geschehen kann. Im zweiten Fall sind die mattierte Endfläche 3 und die Oberfläche der Halterung zu einer Steckverbindung ausgearbeitet, wie es gestrichelt gezeichnet ist. Bisher wurde angenommen, dass die Endfläche 3 mattiert und die Oberfläche der Halterung 4 glatt sind. Selbstverständlich können die Endfläche 3 glatt und die Oberfläche der Halterung 4 mattiert sein. Dies ist dann von Vorteil, wenn die auf der Endfläche 2 liegende Kontaktlinse mit dem Durchlichtverfahren (mattierte Fläche, mit z.B. Lichtquelle 5) oder mit dem Auflichtverfahren (glatte Fläche, z.B. Figuren 1,6) betrachtet wird. Der Körper 1 wird hierzu aus den entsprechenden Elementen 4,8,11 zusammengesetzt. Zweckmässig wird das Element 8 mit der gewünschten Krümmung gewählt.

Die Figur 6 zeigt den transparenten Körper 1 mit glatter Oberfläche, in welchem eine Sammellinse 6 integriert ist. Sie kann wie gezeichnet im Körper 1 oder in einem nicht dargestellten Körperelement 11 angeordnet sein. Im letzteren Falle wurden die entsprechenden Elemente wunschgemäss zusammengesetzt. Die ebene Fläche 3 ist nicht mattiert. Die auf der sphärischen Endfläche 2 befindliche Kontaktlinse wird in der Weise betrachtet, dass die ebene Endfläche 3 dem Auge des Betrachters näher ist als die sphärische Endfläche 2 mit der Kontaktlinse. Die Kontaktlinse wird gegen einen dunklen Hintergrund gehalten. Die als Lupe wirkende Sammellinse 6 vergrössert das Bild der Kontaktlinse. Die Sammellinse 6 wird im transparenten Körper 1 so angeordnet, dass sie von der Kontaktlinse auf der sphärischen Endfläche 2 ein reelles oder virtuelles, vergrössertes Bild erzeugt. Dies hängt davon ab, ob der Abstand Sammellinse 6 - Endfläche 2 grösser oder kleiner als die Brennweite der Sammellinse ist. Die Sammellinse 6 kann auch auf der entsprechend geformten Endfläche 3 angeklebt sein, so dass kein Zwischenraum zwischen beiden besteht. Das Material der Sammellinse 6, das aus einem gasförmigen, flüssigen oder festen Medium bestehen kann, muss einen anderen Brechungsindex haben als das Material des Körpers 1.

Es besteht auch die Möglichkeit, eine in Figur 6 gezeichnete Zerstreuungslinse 7 gemeinsam mit der Sammellinse 6 im stabförmigen Körper 1 vorzusehen oder auf die entsprechend geformte Endfläche 3 ohne Zwischenraum zu kleben. Auch hier ist die Endfläche 3 dem Auge des Betrachters zugewandt; d.h., sie ist dem Auge näher als die Endfläche 2 mit der Kontaktlinse, welche gegen einen dunklen Hintergrund gehalten wird. Das Medium der Zerstreuungslinse 7 hat einen anderen Brechungsindex wie das Material des Körpers 1 und kann von gasförmiger, flüssiger oder fester Konsistenz sein, wie z.B. Luft, Wasser oder ein anderer Glastyp. Der Abstand zwischen den beiden Linsen 6, 7 ist so gewählt, dass das durch die Sammellinse 6 erzeugte Bild der auf der Endfläche 2 befindlichen Kontaktlinse zwischen der einfachen und der doppelten Brennweite der Zerstreuungslinse 7 liegt, was ein vergrössertes virtuelles Bild für den Betrachter der Kontaktlinse ergibt. Der Vorteil der zusätzlichen Zerstreuungslinse 7 ist, dass die bei Sammellinsen mit hohen Dioptriewerten übliche Einschränkung ihrer Bildfläche beseitigt wird.

Die Sammellinse 6 und die Zerstreuungslinse 7 können fest im stabförmigen Körper 1 eingebaut oder in nicht gezeichneten Elementen vorgesehen sein, die zusammengesteckt werden. In beiden Fällen ergeben sich vergrösserte Bilder der Kontaktlinsen. Der stabförmige Körper 1 der Figur 6 oder die Elemente können einen kreisförmigen, elliptischen oder polygonen Querschnitt haben. Anstelle der Linsen kann auch eine Vergrösserungsoptik vorgesehen sein.

Die Figur 7 zeigt den stabförmigen Körper 1, der aus einigen Elementen zusammengesetzt ist, von denen nur zwei gezeichnet sind. Das Element 10 enthält, wie bei den früheren Ausführungsbeispielen der Erfindung, die eine Endfläche 2 und einen Steck-bzw. Schraubverschluss 9. Ferner ist das Element 10 als Deckel ausgebildet und ergibt einen Hohlraum 12 mit der besonders geformten Vertiefung im Körperelement 11. Die Vertiefung läuft flach aus. Im Hohlraum 12 kann nur eine Kontaktlinse aufbewahrt werden, welche in ihrer Position festgehalten wird. Die Aufbewahrung im abgeschlossenen Hohlraum 12 mit einer Aufbewahrungsflüssigkeit ist bei weichen und harten Kontaktlinsen von Vorteil, da diese nicht austrocknen und somit nicht zerstört werden können. Am unteren Teil des Körperelementes 11 ist eine Schraub- oder Steckverbindung 14 für ein weiteres Element vorgesehen, das eine Beleuchtung oder ein Linsenglied enthalten kann. Der in Figur 7 gezeigte Körper kann als Aufbewahrung (Etui) einer Kontaktlinse dienen. Für ein Kontaktlinsenpaar werden 2 Körper benötigt, die sowohl jeder für sich unabhängig als auch mittels einer Halterung miteinander verbunden werden können. Die Halterung kann entweder eine Klippeinrichtung sein, in welche die beiden stabförmigen Körper 1 eingeklippt werden, oder als ein mit den beiden Körpern integriertes Stück ausgebildet sein. Der Vorteil hiervon ist die Kombination eines Kontaktlinsenetuis und einer Ueberwachungsvorrichtung für diese.

Der Körper 1 ist in den Figuren 1 - 7 mit blanker durchsichtiger Seitenfläche beschrieben und gezeigt worden. In vorteilhafter Weise können seine Seitenflächen schwarz gefärbt sein, wodurch sich unliebsame Reflexionen und unerwünschte Abbildungen vermeiden lassen. Wenn z.B. dieser Körper von einer Beleuchtungsquelle seitlich beleuchtet wird, ist die auf der Endfläche 2 angeordnete Kontaktlinse vor einem dunklen, Reflex- und Abbildungsfreien Hintergrund sichtbar.

Die in den Figuren 1 - 7 beschriebenen Ausführungsbeispiele können vom Anpasser (Augenarzt, Optiker) und vom Träger zur Betrachtung von Kontaktlinsen herangezogen werden. Vorteilhaft sind die schnellen und unkomplizierten Vorbereitungen hierzu, da die erfindungsgemässe Vorrichtung schnell zur Hand ist und bei den meisten Ausführungen ohne künstliche Beleuchtung auskommt. Da die Länge und der Durchmesser des Körpers 10 cm und 3 cm nicht überschreiten, ist die erfindungsgemässe Vorrichtung klein, von geringem Gewicht und leicht transportierbar. Ausserdem sind die Herstellungskosten gering. Jeder Kontaktlinsenträger ist nun in der Lage, die Ablagerungen und Materialschäden an seinen Kontaktlinsen so früh zu erkennen, dass ihre Reinigung den gewünschten Erfolg hat und Komplikationen vermindert werden. Dies gilt besonders in den Fällen, in welchen eine Kontaktlinsenpraxis eines Augenarztes oder Optikers nicht in der Nähe oder geschlossen ist.

## Patentansprüche

1. Untersuchungsvorrichtung zum Erkennen von Materialveränderungen an Kontaktlinsen und Ablagerungen auf deren Oberflächen, die während des Tragens der Kontaktlinsen entstehen, welche Untersuchungsvorrichtung gemeinsam mit der Kontaktlinse einer Lichtquelle entgegengehalten und mit dem Auge betrachtet werden kann, **gekennzeichnet durch** einen stabförmigen Körper (1) mit kreisförmigem, elliptischem oder polygonem Querschnitt aus optisch durchsichtigem Material und zwei Endflächen, dessen eine Endfläche (2) wahlweise sphärisch, torisch oder asphärisch und dessen andere Endfläche (3) wahlweise eben, konvex oder konkav ausgebildet sind, wobei die zur Aufnahme der zu untersuchenden Kontaktlinse dienende Endfläche (2) eine Krümmung hat, die im Bereich des Krümmungsradius der Kontaktlinse liegt.

2. Vorrichtung nach Patentanspruch 1, **dadurch gekennzeichnet**, dass die Krümmung der die Kontaktlinse aufnehmenden Endfläche (2) von einem Krümmungsradius oder von mindestens zwei unterschiedlichen Krümmungsradien gebildet ist (Figuren 2, 3, 4).

3. Vorrichtung nach Patentansprüch 1 oder 2, **dadurch gekennzeichnet**, dass von den beiden Endflächen des stabförmigen Körpers (1) die die Kontaktlinse aufnehmende Endfläche (2) transparent ist und eine glatte Oberfläche hat und die andere Endfläche (3) eben und mattiert ist (Figuren 1, 5).

4. Vorrichtung nach Patentanspruch 2, **dadurch gekennzeichnet**, dass an der ebenen und mattierten Endfläche (3) eine Halterung (4) mit einer Beleuchtungsquelle (5) angebracht ist (Figur 5).

5. Vorrichtung nach Patentanspruch 1 oder 2, **dadurch gekennzeichnet,** dass die beiden Endflächen (2,3) des stabförmigen Körpers (1) transparent sind und glatte Oberflächen aufweisen (Figur 6).

6. Vorrichtung nach Patentanspruch 5, **dadurch gekennzeichnet**, dass der stabförmige Körper (1) eine Sammellinse (6) aus einem gasförmigen, flüssigen oder festen Medium enthält (Figur 6).

7. Vorrichtung nach Patentanspruch 5, **dadurch gekennzeichnet,** dass der stabförmige Körper (1) eine Zerstreuungslinse (7) aus einem gasförmigen, flüssigen oder festen Medium enthält (Figur 6).

8. Vorrichtung nach einem der vorhergehenden Patentansprüche, **dadurch gekennzeichnet,** dass der stabförmige Körper (1) und seine beiden Endflächen (2,3) aus einem optisch durchsichtigen Stück bestehen (Figuren 1,2, 4,6).

9. Vorrichtung nach einem der Patentansprüche 1 - 7, **dadurch gekennzeichnet,** dass der stabförmige Körper (1) aus mindestens zwei optisch durchsichtigen Elementen (4,8,9,10,11) zusammensetzbar ist (Figuren 3,5,7).

10. Vorrichtung nach Patentanspruch 9, **dadurch gekennzeichnet,** dass ein Element (8) des stabförmigen Körpers (1) die eine Endfläche (2) mit der gewünschten Krümmung zur Aufnahme der Kontaktlinse enthält (Figuren 3,5).

11. Vorrichtung nach Patentanspruch 9, **dadurch gekennzeichnet,** dass ein anderes Element (4) des stabförmigen Körpers (1) als Halterung für eine Beleuchtungsquelle (5) ausgebildet ist (Figur 5).

12. Vorrichtung nach Patentanspruch 9, **dadurch gekennzeichnet**, dass ein weiteres Element (10) des stabförmigen Körpers (1) als ein durchsichtiger Deckel ausgebildet ist, der mit einem Ele-ment (11) zusammengesetzt einen Hohlraum (12) zur Aufnahme der Kontaktlinse und einer Aufbewahrungsflüssigkeit bildet (Figur 7).

13. Vorrichtung nach Patentanspruch 12, **dadurch gekennzeichnet,** dass ein stabförmiger Körper (1) mittels einer Haltevorrichtung mit einem weiteren stabförmigen Körper verbunden ist (Figur 7).

14. Untersuchungsvorrichtung nach einem der vorherigen Patentansprüche, **dadurch gekennzeichnet**, dass der stabförmige Körper (1) schwarzgefärbte Seitenflächen hat und die auf der Endfläche (2) angeordnete Kontaktlinse gegebenenfalls seitlich von einer Beleuchtungsquelle angestrahlt werden kann.

## Claims

1. Inspection device for detecting material changes on contact lenses and deposits on the surfaces thereof which arise during the wearing of the contact lenses, which inspection device can be held up together with the contact lens against a light source and can be examined by eye, characterised by a rod-shaped member (1) having a circular, elliptical or polygonal cross-section made from an optically transparent material and two end faces, one end face (2) of which is optionally constructed to be spherical, toroidal or aspherical and the other end face (3) of which is optionally constructed to be flat, convex or concave, the end face (2) serving to accommodate the contact lens to be inspected having a curvature which is in the range of the radius of curvature of the contact lens.

2. Device according to Patent Claim 1, characterised in that the curvature of the end face (2) accommodating the contact lens is formed from one radius of curvature or from at least two different radii of curvature (Figures 2, 3, 4).

3. Device according to Patent Claim 1 or 2, characterised in that, of the two end faces of the rod-shaped member (1), the end face (2), accommodating the contact lens, is transparent and has a smooth surface, and the other end face (3) is flat and frosted (Figures 1, 5).

4. Device according to Patent Claim 2, characterised in that a holder (4) having an illumination source (5) is mounted on the flat and frosted end face (3) (Figure 5).

5. Device according to Patent Claim 1 or 2, characterised in that the two end faces (2, 3) of the rod-shaped body (1) are transparent and have smooth surfaces (Figure 6).

6. Device according to Patent Claim 5, characterised in that the rod-shaped member (1) contains a converging lens (6) made from a gaseous, liquid or solid medium (Figure 6).

7. Device according to Patent Claim 5, characterised in that the rod-shaped member (1) contains a diverging lens (7) made from a gaseous, liquid or solid medium (Figure 6).

8. Device according to one of the preceding patent claims, characterised in that the rod-shaped member (1) and its two end faces (2, 3) consist of an optically transparent piece (Figures 1, 2, 4, 6).

9. Device according to one of Patent Claims 1 - 7, characterised in that the rod-shaped member (1) can be assembled from at least two optically transparent elements (4, 8, 9, 10, 11) (Figures 3, 5, 7).

10. Device according to Patent Claim 9, characterised in that an element (8) of the rod-shaped member (1) contains one end face (2) with the desired curvature for accommodating the contact lens (Figures 3, 5).

11. Device according to Patent Claim 9, characterised in that another element (4) of the rod-shaped member (1) is constructed as a holder for an illumination source (5) (Figure 5).

12. Device according to Patent Claim 9, characterised in that a further element (10) of the rod-shaped member (1) is constructed as a transparent cover which, when assembled with an element (11), forms a cavity (12) for accommodating the contact lens and a preserving liquid (Figure 7).

13. Device according to Patent Claim 12, characterised in that a rod-shaped member (1) is connected by means of a holding device to a further rod-shaped member (Figure 7).

14. Inspection device according to one of the preceding patent claims, characterised in that the rod-shaped member (1) has blackened side faces and the contact lens arranged on the end face (2) can be irradiated from the side by an illumination source, if necessary.

## Revendications

1. Dispositif d'examen pour la mise en évidence de modifications du matériau constituant de lentilles de contact et de dépôts sur leurs surfaces, qui se forment lors du port des lentilles de contact, ce dispositif d'examen, en même temps que la lentille de contact, pouvant être tourné vers une source de lumière et observé à l'oeil nu, caractérisé par un corps en forme de barreau (1) de section transversale circulaire, elliptique ou polygonale, en matériau optiquement transparent, et par deux faces d'extrémité, dont l'une (2) peut être sphérique, torique ou asphérique, et dont l'autre (3) peut être plate, convexe ou concave, la face d'extrémité (2) servant à recevoir la lentille de contact à examiner ayant une courbure voisine du rayon de courbure de la lentille de contact.

2. Dispositif suivant la revendication 1, caractérisé en ce que la courbure de la face d'extrémité (2) recevant la lentille de contact est formée par un rayon de courbure ou au moins deux rayons de courbure différents (figures 2, 3, 4).

3. Dispositif suivant la revendication 1 ou 2, caractérisé en ce que, sur les deux faces d'extrémité du corps en forme de barreau (1), la face d'extrémité (2) recevant la lentille de contact est transparente et a une surface lisse, et l'autre face d'extrémité (3) est plate et dépolie (figures 1, 5).

4. Dispositif suivant la revendication 2, caractérisé en ce qu'un support (4) muni d'une source de lumière (5) vient s'appliquer à la face d'extrémité plate et dépolie (3) (figure 5).

5. Dispositif suivant la revendication 1 ou 2, caractérisé en ce que les deux faces d'extrémité (2, 3) du corps en forme de barreau (1) sont transparentes et présentent des surfaces lisses (figure 6).

6. Dispositif suivant la revendication 5, caractérisé en ce que le corps en forme de barreau (1) contient une lentille convergente (6) en milieu gazeux, liquide ou solide (figure 6).

7. Dispositif suivant la revendication 5, caractérisé en ce que le corps en forme de barreau (1) contient une lentille divergente (7) en milieu gazeux, liquide ou solide (figure 6).

8. Dispositif suivant l'une des revendications précédentes, caractérisé en ce que le corps en forme de barreau (1) et ses deux faces d'extrémité (2,3) consistent en un bloc optiquement transparent (figures 1, 2, 4, 6).

9. Dispositif suivant l'une des revendications 1 à 7, caractérisé en ce que le corps en forme de barreau (1) peut être constitué par l'assemblage d'au moins deux éléments optiquement transparents (4, 8, 9, 10, 11) (figures 3, 5, 7).

10. Dispositif suivant la revendication 9, caractérisé en ce qu'un élément (8) du corps en forme de barreau (1) contient la face d'extrémité (2) qui a la courbure requise pour recevoir la lentille de contact (figures 3, 5).

11. Dispositif suivant la revendication 9, caractérisé en ce qu'un autre élément (4) du corps en forme de barreau (1) est concu en tant que support pour une source de lumière (5) (figure 5).

12. Dispositif suivant la revendication 9, caractérisé en ce qu'un autre élément (10) du corps en forme de barreau (1) est concu en tant que calotte transparente, qui, assemblée à un élément (11), forme une cavité (12) pour recevoir la lentille de contact et un liquide de conservation (figure 7).

13. Dispositif suivant la revendication 12, caractérisé en ce qu'un corps en forme de barreau (1) est connecté à un autre corps en forme de barreau au moyen d'un dispositif de maintien (figure 7).

14. Dispositif suivant l'une des revendications précédentes, caractérisé en ce que le corps en forme de barreau (1) a des faces latérales peintes de couleur noire et en ce que la lentille de contact disposée sur la face d'extrémité (2) peut, le cas échéant, être éclairée latéralement par une source de lumière.
